(19) Europäisches Patentamt
European Patent Office
Office européen des brevets

(11) Veröffentlichungsnummer: **0 451 653 A2**

# (12) EUROPÄISCHE PATENTANMELDUNG

(21) Anmeldenummer: **91105114.2**

(22) Anmeldetag: **30.03.91**

(51) Int. Cl.[5]: **C07D 239/60**, C07D 405/12, C07D 239/52, C07D 401/12, A01N 43/54

(30) Priorität: **11.04.90 DE 4011696**
**24.10.90 DE 4033808**

(43) Veröffentlichungstag der Anmeldung:
**16.10.91 Patentblatt 91/42**

(84) Benannte Vertragsstaaten:
**BE CH DE ES FR GB IT LI NL**

(71) Anmelder: **BAYER AG**

**W-5090 Leverkusen 1 Bayerwerk(DE)**

(72) Erfinder: **Drewes, Mark Wilhelm, Dr.**
**Gruenstrasse 30**
**W-4018 Langenfeld(DE)**
Erfinder: **Haug, Michael, Dr.**
**Fahner Weg 5**
**W-5060 Bergisch Gladbach 2(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Gruenstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

## (54) Naphthalinderivate.

(57) Die Erfindung betrifft neue Naphthalinderivate der allgemeinen Formel (I),

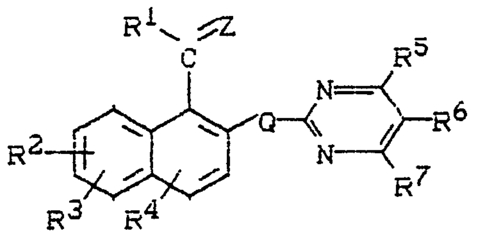

(I)

in welcher
Q für Sauerstoff, Schwefel oder die Gruppierung NR steht, wobei R für Wasserstoff oder Alkyl steht,
$R^1$ für Wasserstoff oder Alkyl steht und
Z für eine der nachstehenden Gruppierungen steht: N-$R^8$ oder

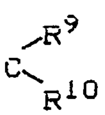

,

(wobei die Reste $R^2$ - $R^{10}$ die in der Beschreibung angegebenen Bedeutungen haben), Verfahren zu ihrer

EP 0 451 653 A2

Herstellung und ihre Verwendung als Herbizide.

Die Erfindung betrifft neue Naphthalinderivate, Verfahren zu ihrer Herstellung und ihre Verwendung als Herbizide.

Es ist bereits bekannt, daß bestimmte Naphthalinderivate, wie z.B. 2-(4,6-Dimethoxy-pyrimidin-2-yl-oxy)-1-naphthaldehyd, herbizide Eigenschaften aufweisen (vgl. EP-A 360 163; JP-A 2 056 469).

Die herbizide Wirksamkeit der bekannten Naphthalinderivate und ihre Verträglichkeit gegenüber Nutzpflanzen ist jedoch nicht immer ganz zufriedenstellend.

Es wurden nun die neuen Naphthalinderivate der allgemeinen Formel (I) gefunden,

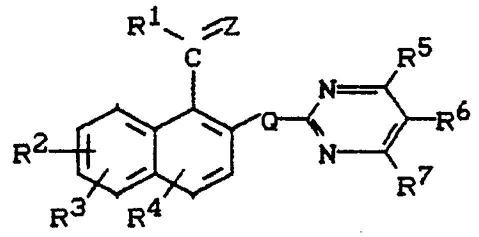

(I)

in welcher

| | |
|---|---|
| Q | für Sauerstoff, Schwefel oder die Gruppierung NR steht, wobei R für Wasserstoff oder Alkyl steht, |
| $R^1$ | für Wasserstoff oder Alkyl steht, |
| $R^2$ $R^3$ und $R^4$ | gleich oder verschieden sind und jeweils für Wasserstoff, Hydroxy, Amino, Cyano, Nitro, Halogen oder für jeweils gegebenenfalls substituiertes Alkyl, Alkoxy, Alkylthio, Alkylsulfinyl oder Alkylsulfonyl stehen, |
| $R^5$ | für Wasserstoff, Halogen oder für jeweils gegebenenfalls substituiertes Alkyl, Alkoxy, Alkylthio, Alkylamino oder Dialkylamino steht, |
| $R^6$ | für Wasserstoff, Halogen oder für jeweils gegebenenfalls substituiertes Alkyl oder Alkoxy steht und |
| $R^7$ | für Wasserstoff, Halogen oder für jeweils gegebenenfalls substituiertes Alkyl, Alkoxy, Alkylthio, Alkylamino oder Dialkylamino steht, und |
| Z | für eine der nachstehenden Gruppierungen steht: N-$R^8$ oder |

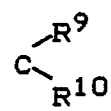

,

wobei

| | |
|---|---|
| $R^8$ | für Wasserstoff, Hydroxy, Amino, Carbamoylamino oder für jeweils gegebenenfalls substituiertes Alkyl, Alkenyl, Alkinyl, Alkoxy, Alkenyloxy, Alkoxycarbonylalkoxy, Alkylamino, Dialkylamino, Alkylcarbonylamino, Alkoxycarbonylamino, Alkylsulfonylamino, Aryl, Aralkyl, Aryloxy, Aralkyloxy, Arylamino, Diarylamino, Aralkylamino, N-Alkyl-N-arylamino, Hetarylamino, Hetarylcarbonylamino, Arylcarbonylamino oder Arylsulfonylamino steht, |
| $R^9$ | für Wasserstoff, Halogen, Cyano, Carboxy, Alkoxycarbonyl, Alkylcarbonylamino oder Dialkoxyphosphoryl steht und |
| $R^{10}$ | für Formyl, Cyano, Carboxy, Hydroxymethyl, Carbamoyl oder für jeweils gegebenenfalls substituiertes Alkoxycarbonyl, Cycloalkoxycarbonyl, Alkylthiocarbonyl, Alkylaminocarbonyl, Cycloalkylaminocarbonyl, Dialkylaminocarbonyl, Alkylaminocarbonylalkoxycarbonyl, Dialkylaminocarbonylalkoxycarbonyl, Arylaminocarbonylalkoxycarbonyl, N-Alkyl-N-arylaminocarbonylalkoxycarbonyl, Pyrrolidinylcarbonyl, Piperidinylcarbonyl, Morpholinylcarbonyl, Piperazinylcarbonyl, Aryloxycarbonyl, Aralkyloxycarbonyl, Heterocyclylalkoxycarbonyl, Arylthiocarbonyl, Aralkylthiocarbonyl, Arylaminocarbonyl, Aralkylaminocarbonyl, N-Alkyl-N-arylaminocarbonyl, Arylhydrazinocarbonyl, Alkylhydrazinocarbonyl oder Phthalimidoxycarbonyl steht oder $R^{10}$ zusammen mit $R^9$ für die Gruppierung $-CO-O-(CH_2)_n-$ steht, wobei n für die Zahlen 1 bis 4 steht. |

Man erhält die neuen Naphthalinderivate der allgemeinen Formel (I), wenn man

(a) entsprechende Carbonylverbindungen der allgemeinen Formel (II)

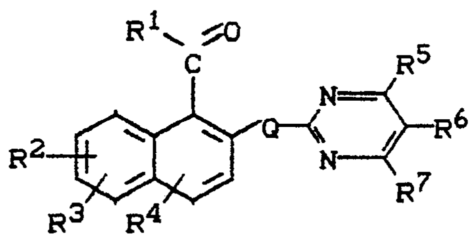

(II)

in welcher

Q, $R^1$, $R^2$, $R^3$, $R^4$, $R^5$, $R^6$ und $R^7$ die oben angegebene Bedeutung haben,

mit Amino- oder Methylen-Verbindungen der allgemeinen Formel (III)

$H_2Z$ (III)

in welcher

Z die oben angegebene Bedeutung hat,

gegebenenfalls in Gegenwart eines Reaktionshilfsmittels und gegebenenfalls in Gegenwart eines Verdünnungsmittels umsetzt, oder wenn man

(b) nucleophile Naphthalinderivate der allgemeinen Formel (IV)

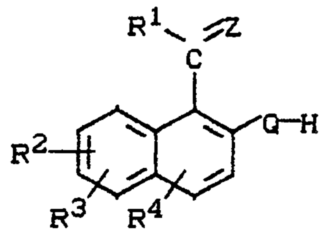

(IV)

in welcher

Q, $R^1$, $R^2$, $R^3$, $R^4$ und Z die oben angegebene Bedeutung haben,

mit Pyrimidinderivaten der allgemeinen Formel (V)

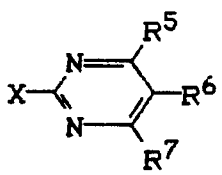

(V)

in welcher

$R^5$, $R^6$ und $R^7$ die oben angegebene Bedeutung haben und

X für eine nucleofuge Abgangsgruppe steht,

gegebenenfalls in Gegenwart eines Säureakzeptors und gegebenenfalls in Gegenwart eines Verdünnungsmittels umsetzt.

Die neuen Naphthalinderivate der allgemeinen Formel (I) zeichnen sich durch starke Herbizidwirkung aus.

Überraschenderweise zeigen die erfindungsgemäßen Naphthalinderivate der Formel (I) bei guter Verträglichkeit gegenüber wichtigen Kulturpflanzen wesentlich stärkere Wirkung gegen Unkräuter als die bekannte Verbindung 2-(4,6-Dimethoxy-pyrimidin-2-yl-oxy)-1-naphthaldehyd, welches ein strukturell ähnlicher vorbekannter Wirkstoff gleicher Wirkungsrichtung ist.

Die Erfindung betrifft vorzugsweise Verbindungen der Formel (I), in welcher

Q für Sauerstoff, Schwefel oder die Gruppierung NR steht, wobei R für Wasserstoff oder $C_1$-$C_4$-Alkyl steht,

$R^1$ für Wasserstoff oder $C_1$-$C_4$-Alkyl steht,

$R^2$, $R^3$ und $R^4$ gleich oder verschieden sind und jeweils für Wasserstoff, Hydroxy, Amino, Cyano, Nitro, Halogen oder für jeweils gegebenenfalls durch Halogen substituiertes $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl stehen,

$R^5$ für Wasserstoff, Halogen oder für jeweils gegebenenfalls durch Halogen oder $C_1$-$C_4$-Alkoxy substituiertes $C_1$-$C_4$-Alkyl, $C_1$-$C_4$-Alkoxy, $C_1$-$C_4$-Alkylthio, $C_1$-$C_4$-Alkylamino oder Di-($C_1$-$C_4$-alkyl)-amino steht,

$R^6$ für Wasserstoff, Halogen oder für jeweils gegebenenfalls durch Halogen substituiertes $C_1$-$C_4$-Alkyl oder $C_1$-$C_4$-Alkoxy steht,

$R^7$ für Wasserstoff, Halogen oder für jeweils gegebenenfalls durch Halogen substituiertes $C_1$-$C_4$-Alkyl, $C_1$-$C_4$-Alkoxy, $C_1$-$C_4$-Alkylthio, $C_1$-$C_4$-Alkylamino oder Di-($C_1$-$C_4$-alkyl)-amino steht,

Z für eine der nachstehenden Gruppierungen steht: N-$R^8$ oder

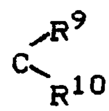 ,

wobei

$R^8$ für Wasserstoff, Hydroxy, Amino, Carbamoylamino oder für jeweils gegebenenfalls durch Halogen substituiertes $C_1$-$C_6$-Alkyl, $C_3$-$C_6$-Alkenyl, $C_3$-$C_6$-Alkinyl, $C_1$-$C_6$-Alkoxy, $C_3$-$C_6$-Alkenyloxy, $C_1$-$C_4$-Alkoxy-carbonyl-$C_1$-$C_2$-alkoxy, $C_1$-$C_6$-Alkylamino, Di-($C_1$-$C_2$-alkyl)-amino, $C_1$-$C_6$-Alkylcarbonylamino, $C_1$-$C_6$-Alkoxy-carbonylamino, $C_1$-$C_6$-Alkyl-sulfonylamino, oder für jeweils gegebenenfalls durch Nitro, Hydroxy, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituiertes Phenyl, Phenyl-$C_1$-$C_4$-alkyl, Phenoxy, Phenyl-$C_1$-$C_4$-alkoxy, Phenylamino, Diphenylamino, Phenyl-$C_1$-$C_4$-alkylamino, N-($C_1$-$C_4$-alkyl)-N-phenylamino, Pyridylamino, Pyrimidylamino, Chinolylamino, Pyridylcarbonylamino, Phenylcarbonylamino, Furylcarbonylamino, Thienylcarbonylamino oder Phenylsulfonylamino steht,

$R^9$ für Wasserstoff, Halogen, Cyano, Carboxy, $C_1$-$C_6$-Alkoxy-carbonyl, $C_1$-$C_6$-Alkylcarbonylamino oder Di-($C_1$-$C_4$-alkoxy)-phosphoryl steht und

$R^{10}$ für Formyl, Cyano, Carboxy, Hydroxymethyl, Carbamoyl oder für jeweils gegebenenfalls durch Halogen, Carboxy oder $C_1$-$C_4$-Alkoxy-carbonyl substituiertes $C_1$-$C_6$-Alkoxy-carbonyl, $C_5$-$C_6$-Cycloalkyloxy-carbonyl, $C_1$-$C_6$-Alkylthiocarbonyl, $C_1$-$C_6$-Alkylamino-carbonyl oder $C_5$-$C_6$-Cycloalkylamino-carbonyl, für Di-($C_1$-$C_2$-alkyl)-amino-carbonyl, für $C_1$-$C_4$-Alkylamino-carbonyl-$C_1$-$C_4$-alkoxy-carbonyl, für Di-($C_1$-$C_2$-alkyl)-amino-carbonyl-$C_1$-$C_4$-alkoxy-carbonyl, für Phenylaminocarbonyl-$C_1$-$C_4$-alkoxy-carbonyl, für N-Methyl-N-phenylaminocarbonyl-$C_1$-$C_4$-alkoxycarbonyl, für jeweils gegebenenfalls durch Methyl und/oder Ethyl substituiertes Pyrrolidinylcarbonyl, Piperidinyl-carbonyl, Morpholinylcarbonyl oder Piperazinylcarbonyl, für jeweils gegebenenfalls durch Nitro, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)amino substituiertes Phenoxycarbonyl, Phenyl-$C_1$-$C_4$-alkoxycarbonyl, Furylmethoxycarbonyl, Thienylmethoxycarbonyl, Phenylthiocarbonyl, Phenyl-$C_1$-$C_4$-alkylthio-carbonyl, Phenylaminocarbonyl, Phenyl-$C_1$-$C_4$-alkylamino-carbonyl, N-($C_1$-$C_4$-Alkyl)-N-phenylamino-carbonyl oder Phenylhydrazinocarbonyl, für $C_1$-$C_4$-Alkylhydrazinocarbonyl oder für Phthalimidoxycarbonyl steht oder $R^{10}$ zusammen mit $R^9$ für die Gruppierung -CO-O-$(CH_2)_n$-steht, wobei

n für die Zahlen 1 bis 4, insbesondere 2 oder 3 steht.

Die in der Definition der erfindungsgemäßen Verbindungen aufgeführten aliphatischen Kohlenwasserstoffreste (z.B. Alkyl, Alkenyl, Alkinyl), auch in Kombination mit Heteroatomen (z.B. in Alkoxy, Alkylthio, Alkylamino) oder in Zusammensetzungen wie z.B. Halogenalkyl, Halogenalkoxy, sind jeweils geradkettig oder verzweigt.

Halogen steht im allgemeinen für Fluor, Chlor, Brom oder Jod, vorzugsweise für Fluor, Chlor oder Brom, insbesondere für Fluor oder Chlor.

Die Erfindung betrifft insbesondere Verbindungen der Formel (1), in welcher

Q für Sauerstoff steht,

$R^1$ für Wasserstoff steht,

$R^2$, $R^3$ und $R^4$ für Wasserstoff stehen,

$R^5$ für Wasserstoff, Chlor, Methyl, Ethyl, Trifluormethyl, Methoxymethyl, Methoxy, Ethoxy, Difluormethoxy, Methylthio, Methylamino, Ethylamino oder Dimethylamino - insbesondere für Methoxy - steht,

$R^6$ für Wasserstoff, Chlor, Methyl oder Methoxy - insbesondere für Wasserstoff - steht, und

$R^7$ für Wasserstoff, Chlor, Methyl, Ethyl, Trifluormethyl, Methoxy, Ethoxy, Difluormethoxy, Methylthio, Methylamino, Ethylamino oder Dimethylamino - insbesondere für Methoxy - steht, und

Z für eine der nachstehenden Gruppierungen steht:

N-$R^8$ oder

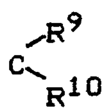 ,

wobei

$R^8$ für Wasserstoff, Hydroxy, Amino, Carbamoylamino, für Methyl, Ethyl, n-Propyl, Isopropyl, n-Butyl, Isobutyl, sec-Butyl, tert-Butyl, Allyl, Propargyl, Methoxy, Ethoxy, n-Propoxy, Isopropoxy, n-Butoxy, Isobutoxy, sec-Butoxy, Allyloxy, Methoxycarbonylmethoxy, Ethoxycarbonylmethoxy, Methoxycarbonylethoxy, Ethoxycarbonylethoxy, Methylamino, Ethylamino, n-Propylamino, Isopropylamino, n-Butylamino, Isobutylamino, sec-Butylamino, tert-Butylamino, Dimethylamino, Acetylamino, Propionylamino, Methoxycarbonylamino, Ethoxycarbonylamino, Methylsulfonylamino, Ethylsulfonylamino, für jeweils gegebenenfalls durch Nitro, Hydroxy, Cyano, Carboxy, Fluor, Chlor, Brom, Methyl, Trifluormethyl, Methoxy, Trifluormethoxy, Methylthio oder Trifluormethylthio substituiertes Phenyl, Benzyl, Phenoxy, Benzyloxy, Phenylamino, Diphenylamino, Benzylamino, N-Methyl-N-phenylamino, Pyridylamino, Pyrimidylamino, Chinolylamino, Pyridylcarbonylamino, Phenylcarbonylamino, Furylcarbonylamino, Thienylcarbonylamino oder Phenylsulfonylamino steht,

$R^9$ für Wasserstoff, Fluor, Chlor, Cyano, Carboxy, $C_1$-$C_4$-Alkoxy-carbonyl, $C_1$-$C_4$-Alkylcarbonylamino, Dimethoxyphosphoryl oder Diethoxyphosphoryl steht und

$R^{10}$ für Formyl, Cyano, Carboxy, Hydroxymethyl, Carbamoyl, für jeweils gegebenenfalls durch Fluor, Chlor, Carboxy oder $C_1$-$C_4$-Alkoxy-carbonyl substituiertes $C_1$-$C_4$-Alkoxy-carbonyl, $C_5$-$C_6$-Cycloalkyloxy-carbonyl, $C_1$-$C_4$-Alkylthiocarbonyl, $C_1$-$C_4$-Alkylamino-carbonyl oder $C_5$-$C_6$-Cycloalkylamino-carbonyl, für Dimethylaminocarbonyl, für $C_1$-$C_4$-Alkylaminocarbonyl-$C_1$-$C_4$-alkoxy-carbonyl, für Dimethylaminocarbonyl-$C_1$-$C_4$-alkoxy-carbonyl, für N-Methyl-N-phenylaminocarbonyl-$C_1$-$C_4$-alkoxy-carbonyl, für jeweils gegebenenfalls durch Methyl und/oder Ethyl substituiertes Pyrrolidinyl-carbonyl, Piperidinyl-carbonyl, Morpholinyl-carbonyl oder Piperazinyl-carbonyl, für jeweils gegebenenfalls durch Nitro, Cyano, Fluor, Chlor, Brom, Methyl, Trifluormethyl, Methoxy, Trifluormethoxy, Methylthio oder Trifluormethylthio substituiertes Phenoxycarbonyl, Benzyloxycarbonyl, Phenylthiocarbonyl, Benzylthiocarbonyl, Phenylaminocarbonyl, Benzylaminocarbonyl, N-Methyl-N-phenylaminocarbonyl, Phenylhydrazinocarbonyl oder für Phthalimidoxycarbonyl steht oder $R^{10}$ zusammen mit $R^9$ für die Gruppierung $-CO-O-CH_2CH_2-$ steht.

Verwendet man für das erfindungsgemäße Verfahren (a) beispielsweise 2-(4,6-Dimethoxy-pyrimidin-2-yl-oxy)-1-naphthaldehyd und Acethydrazid als Ausgangsstoffe, so kann der Reaktionsablauf durch das folgende Formelschema wiedergegeben werden:

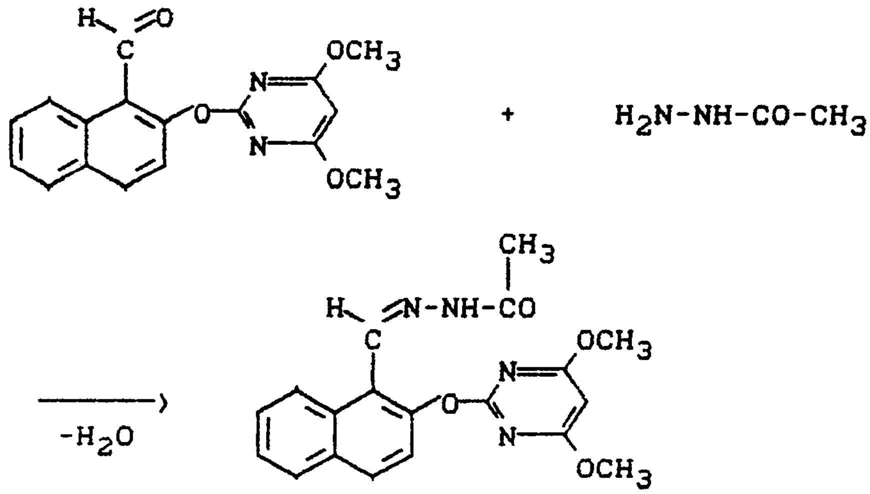

Verwendet man für das erfindungsgemäße Verfahren (b) beispielsweise 1-Methoximinomethyl-2-naphthol und 2-Chlor-4,6-dimethoxy-pyrimidin als Ausgangsstoffe, so kann der Reaktionsablauf durch das folgende Formelschema wiedergegeben werden:

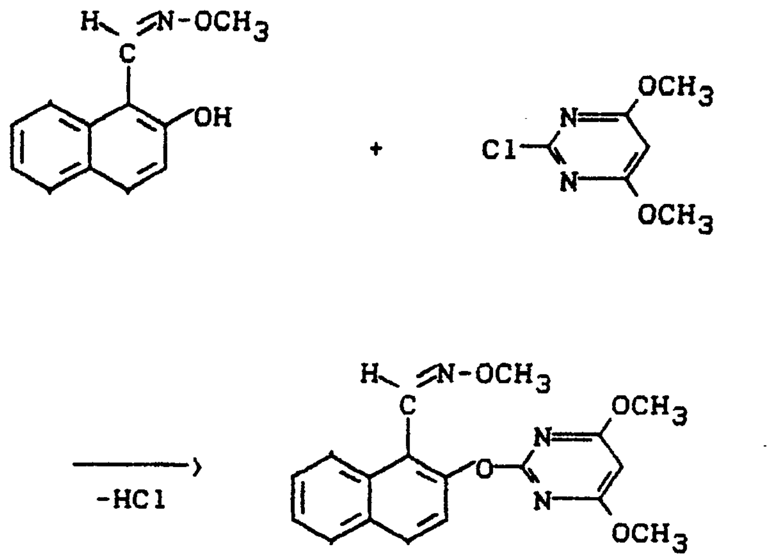

Die beim erfindungsgemäßen Verfahren (a) zur Herstellung von Verbindungen der Formel (I) als Ausgangsstoffe zu verwendenden Carbonylverbindungen sind durch die Formel (II) allgemein definiert.

In Formel (II) haben Q, $R^1$, $R^2$, $R^3$, $R^4$, $R^5$, $R^6$ und $R^7$ vorzugsweise bzw. insbesondere diejenigen Bedeutungen, die bereits oben im Zusammenhang mit der Beschreibung der erfindungsgemäßen Verbindungen der Formel (1) vorzugsweise bzw. als insbesondere bevorzugt für Q, $R^1$, $R^2$, $R^3$, $R^4$, $R^5$, $R^6$ und $R^7$ angegeben wurden.

Als Beispiele für die Ausgangsstoffe der Formel (II) seien genannt: 2-(4,6-Dimethoxy-pyrimidin-2-yl-oxy)-, 2-(4-Methoxy-6-methyl-pyrimidin-2-yl-oxy)-, 2-(4,6-Dimethyl-pyrimidin-2-yl-oxy)-, 2-(4-Methoxy-6-trifluormethyl-pyrimidin-2-yl-oxy)-, 2-(4-Ethyl-6-methoxy-pyrimidin-2-yl-oxy)-, 2-(4-Ethoxy-6-methyl-pyrimidin-2-yl-oxy)- und 2-(4,6-Diethoxy-pyrimidin-2-yl-oxy)-1-naphthaldehyd.

Die Carbonylverbindungen der Formel (II) sind bekannt und/oder können nach an sich bekannten Verfahren hergestellt werden (vgl. EP-A 360 163; JP-A 2 056 469 - zitiert in Chem. Abstracts 113 (1990), 54345m; Herstellungsbeispiele).

Die beim erfindungsgemäßen Verfahren (a) zur Herstellung von Verbindungen der Formel (I) weiter als

Ausgangsstoffe zu verwendenden Amino- oder Methylen-Verbindungen sind durch die Formel (III) allgemein definiert.

In Formel (III) hat Z vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für Z angegeben wurde.

Als Beispiele für die Ausgangsstoffe der Formel (III) seien genannt:

Ammoniak, Hydroxylamin, Hydrazin, Methylamin, Ethylamin, Propylamin, Isopropylamin, Butylamin, Isobutylamin, sec-Butylamin, tert-Butylamin, Allylamin, Propargylamin, O-Methyl-, O-Ethyl-, O-Propyl-, O-Isopropyl-, O-Butyl-, O-Isobutyl- und O-sec-Butyl-hydroxylamin, O-Allyl-hydroxylamin, Aminooxyessigsäure-methylester und -ethylester, α-Aminooxy-propionsäure-methylester und -ethylester, Methylhydrazin, Ethylhydrazin, Propylhydrazin, Isopropylhydrazin, Butylhydrazin, Isobutylhydrazin, sec-Butyl-hydrazin, tert-Butylhydrazin, N,N-Dimethylhydrazin, Acethydrazid, Propionylhydrazid, Methoxycarbonylhydrazin, Ethoxycarbonylhydrazin, Methylsulfonylhydrazin, Ethylsulfonylhydrazin, Phenylhydrazin, Benzoylhydrazin, Benzolsulfonsäurehydrazid, p-Toluolsulfonsäurehydrazid, Malonsäure, Cyanessigsäure, Malonsäuredinitril, Cyanessigsäure-methylester und -ethylester, Malonsäure-dimethylester und -diethylester, γ-Butyrolacton.

Die Ausgangsstoffe der Formel (III) sind bekannte Synthesechemikalien.

Das erfindungsgemäße Verfahren (a) zur Herstellung der neuen Naphthalinderivate der Formel (I) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Das erfindungsgemäße Verfahren (a) wird gegebenenfalls in Gegenwart eines Reaktionshilfsmittels durchgeführt. Geeignete Reaktionshilfsmittel sind Stoffe, die üblicherweise zur Steuerung und/oder Beschleunigung von Kondensationsreaktionen zwischen Carbonylverbindungen und Amino- oder Methylen-Verbindungen verwendet werden. Hierzu gehören insbesondere Stickstoffverbindungen, wie z.B. Ammoniumacetat, β-Alanin, Pyridin und Piperidin.

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 10 °C und 120 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel, gegebenenfalls in Gegenwart eines Reaktionshilfsmittels durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vergleiche die Herstellungsbeispiele).

Die beim erfindungsgemäßen Verfahren (b) zur Herstellung von Verbindungen der Formel (I) als Ausgangsstoffe zu verwendenden nucleophilen Naphthalinderivate sind durch die Formel (IV) allgemein definiert.

In Formel (IV) haben Q, $R^1$, $R^2$, $R^3$, $R^4$ und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für Q, $R^1$, $R^2$, $R^3$, $R^4$ und Z angegeben wurden.

Beispiele für die Ausgangsstoffe der Formel (IV) sind in der nachstehenden Tabelle 1 aufgeführt.

<u>Tabelle 1:</u> Beispiele für die Ausgangsstoffe der Formel (IV)

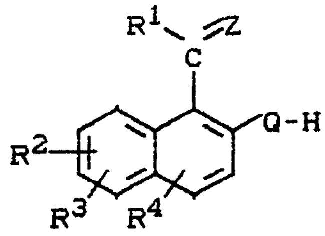

(IV)

| Q | $R^1$ | $R^2$ | $R^3$ | $R^4$ | Z |
|---|---|---|---|---|---|
| O | H | H | H | H | $NOCH_3$ |
| O | H | H | H | H | $NOC_2H_5$ |
| O | H | H | H | H | $NCH_3$ |
| O | H | H | H | H | $NC_2H_5$ |
| O | H | H | H | H | $NCH(CH_3)_2$ |

Tabelle 1 - Fortsetzung

| Q | $R^1$ | $R^2$ | $R^3$ | $R^4$ | Z |
|---|---|---|---|---|---|
| O | H | H | H | H | $NOCH_2COOC_2H_5$ |
| O | H | H | H | H | $NCH_2COOC_2H_5$ |
| O | H | H | H | H | $NNHCOCH_3$ |
| O | H | H | H | H | $NNHSO_2CH_3$ |
| O | H | H | H | H | $NNHCOOCH_3$ |
| O | H | H | H | H | NNH 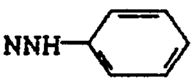 |
| O | H | H | H | H | NNHCO 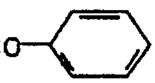 |
| O | H | H | H | H | NNH-$SO_2$ 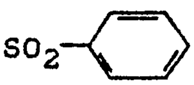 |
| O | H | H | H | H | NNH 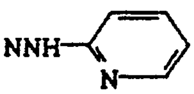 |
| O | H | H | H | H | NNH-CO 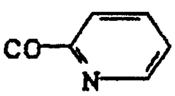 |
| O | H | H | H | H | $NOCH_2$ 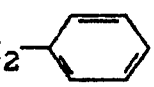 |
| O | H | H | H | H | NNH-CO 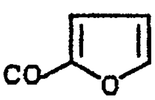 |

**Tabelle 1 - Fortsetzung**

| Q | $R^1$ | $R^2$ | $R^3$ | $R^4$ | Z |
|---|---|---|---|---|---|
| O | H | H | H | H | 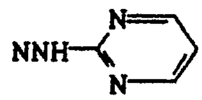 |
| O | H | H | H | H | 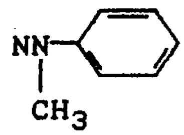 |
| O | H | H | H | H | 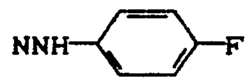 |

Die Ausgangsstoffe der Formel (IV) sind bekannt und/oder können nach an sich bekannten Verfahren hergestellt werden (vgl. Synthesis 1985, 201-202; US-P 4 277 500; Tetrahedron Lett. 1975, 785-788; Chem. Pharm. Bull. 24 (1976), 3065-3074; US-P 4 334 015; Herstellungsbeispiele).

Die beim erfindungsgemäßen Verfahren (b) zur Herstellung von Verbindungen der Formel (I) weiter als Ausgangsstoffe zu verwendenden Pyrimidinderivate sind durch die Formel (V) allgemein definiert.

In Formel (V) haben $R^5$, $R^6$ und $R^7$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^5$, $R^6$ und $R^7$ angegeben wurden und

X steht vorzugsweise für Fluor, Chlor, Brom oder $C_1$-$C_4$-Alkylsulfonyl, insbesondere für Chlor oder Methylsulfonyl.

Als Beispiele für die Ausgangsstoffe der Formel (V) seien genannt:

2-Chlor- und 2-Methylsulfonyl-4,6-dimethyl-pyrimidin, -4-methyl-6-methoxy-pyrimidin, -4,6-dimethoxy-pyrimidin, -4-methyl-6-ethoxy-pyrimidin, -4-chlor-6-methoxy-pyrimidin, -4-methyl-pyrimidin, -4-chlor-6-methyl-pyrimidin, -4-trifluormethyl-6-methoxy-pyrimidin, -4-methoxy-6-difluormethoxy-pyrimidin, -4-methyl-6-difluormethoxy-pyrimidin, -4,6-bis-difluormethoxy-pyrimidin, -4-chlor-6-ethoxy-pyrimidin, -4,6-diethoxy-pyrimidin, -4,5-dichlor-6-methyl-pyrimidin, -4-methyl-5-chlor-6-methoxy-pyrimidin, -4,6-dichlor-pyrimidin, -4-ethyl-6-methoxy-pyrimidin, -5-chlor-4,6-dimethoxy-pyrimidin, -4-methoxy-6-methylamino-pyrimidin sowie -4,6-bis-trifluormethyl-pyrimidin.

Die Pyrimidinderivate der Formel (V) sind bekannt und/oder können nach an sich bekannten Verfahren hergestellt werden (vgl. J. Chem. Soc. 1957, 1830, 1833; J. Org. Chem. 26 (1961), 792; US-P 3 308 119 und US-P 4 711 959).

Das erfindungsgemäße Verfahren (b) zur Herstellung der neuen Naphthalinderivate der Formel (I) wird vorzugsweise unter Verwendung von Verdünnungsmitteln durchgeführt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (b) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (b) in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliohe Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage. Ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); Chloressigsäure-N-(methoxymethyl)-2,6-diethylanilid (ALACHLOR); Methyl-6,6-dimethyl-2,4-dioxo-3-[1-(2-propenyloxyamino)-butyliden]-cyclohexancarbonsäure (ALLOXYDIM); 2-Chlor-4-ethylamino-6-isopropylamino-1,3,5-triazin (ATRAZIN); 3-Isopropyl-2,1,3-benzothiadiazin-4-on-2,2-dioxid (BENTAZON); 3,5-Dibrom-4-hydroxybenzonitril (BROMOXYNIL); N-(3-Chlorphenyl)-isopropylcarbamat (CHLORPROPHAM); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure. (CLOPYRALID); 2-Chlor-4-ethylamino-6-(3-cyanopropylamino)-1,3,5-triazin (CYANAZIN); 2-[1-(Ethoximino)-butyl]-3-hydroxy-5-[tetrahydro-(2H)-thiopyran-3-yl]-2-cyclohexen-1-on (CYCLOXYDIM); 2-[4-(2,4-Dichlorphenoxy)-phenoxy]-propionsäure, deren Methyl-oder deren Ethylester (DICLOFOP); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxyl-phenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOP); [(4-Amino-3,5-dichlor-6-fluor-2-pyridinyl)-oxy]-essigsäure bzw. deren 1-Methylheptylester (FLUROXYPYR); 2-{4-[(3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); Isopropyl-N-phenyl-carbamat (PROPHAM); O-(6-Chlor-3-phenyl-pyridazin-4-yl)-S-octyl-thiocarbonat (PYRIDATE); 2-[4-(6-Chlor-chinoxalin-2-yl-oxy)-phenoxy]-propionsäure-ethylester (QUIZALOFOPETHYL); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); S-[(4-Chlorphenyl)-methyl]-N,N-diethyl-thiocarbamat (THIOBENCARB); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

Herstellungsbeispiele:

Beispiel 1

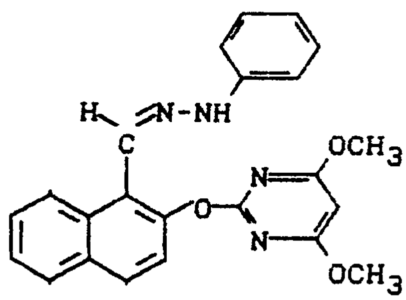

(Verfahren (a))

Eine Mischung aus 3,1 g (10 mmol) 2-(4,6-Dimethoxy-pyrimidin-2-yl-oxy)-1-naphthaldehyd, 1,2 g (10 mmol) Phenylhydrazin und 40 ml Toluol wird bei 20 °C gerührt und anschließend eingeengt. Der Rückstand wird mit Ethanol zur Kristallisation gebracht und das kristalline Produkt durch Absaugen isoliert.

Man erhält 2,2 g (55% der Theorie) 2-(4,6-Dimethoxypyrimidin-2-yl-oxy)-1-naphthaldehyd-phenylhydrazon vom Schmelzpunkt 148 °C.

Beispiel 2

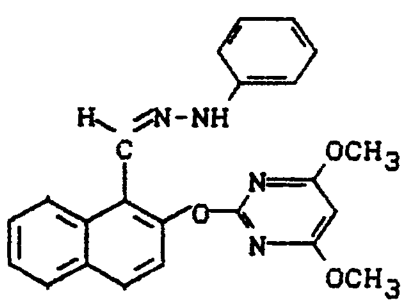

(Verfahren (b))

Zu 4,6-Dimethoxy-2-methylsulfonylpyrimidin (2,2 g, 10 mmol) in Acetonitril (50 ml) gibt man 2-Hydroxy-1-naphthaldedyd-phenylhydrazon (2,6 g, 10 mmol) sowie Kaliumcarbonat (1,4 g). Es wird 7 h unter Rückfluß zum Sieden erhitzt, in Eiswasser gegossen und mit Diethylether extrahiert. Nach dem Trocknen über Natriumsulfat wird eingeengt.

Man erhält 3,1 g (76% der Theorie) 2-(4,6-Dimethoxypyrimidin-2-yl-oxy)-1-naphthaldehyd-phenylhydrazon vom Schmelzpunkt 148° C.

Analog zu den Beispielen 1 und 2 sowie entsprechend der allgemeinen Beschreibung der erfindungsgemäßen Herstellungsverfahren können beispielsweise auch die in der nachstehenden Tabelle 2 aufgeführten Verbindungen der Formel (I) hergestellt werden.

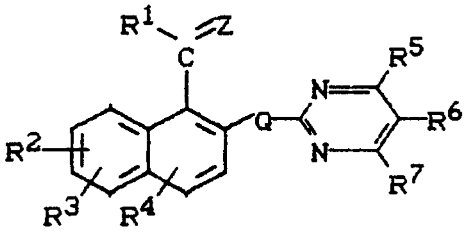

(I)

**Tabelle 2:** Beispiele für die Verbindungen der Formel (I)

| Bsp.-Nr | Q | $R^1$ | $R^2$ | $R^3$ | $R^4$ | $R^5$ | $R^6$ | $R^7$ | Z | Schmelzpunkt (°C) |
|---|---|---|---|---|---|---|---|---|---|---|
| 3 | O | H | H | H | H | $OCH_3$ | H | $OCH_3$ | 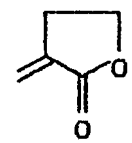 | 129 |
| 4 | O | H | H | H | H | $OCH_3$ | H | $OCH_3$ | CHCOOH | (amorph) |
| 5 | O | H | H | H | H | $OCH_3$ | H | $OCH_3$ | $NNHCH_3$ | (amorph) |
| 6 | O | H | H | H | H | $OCH_3$ | H | $OCH_3$ | $C(CN)_2$ | 118 |
| 7 | O | H | H | H | H | $OCH_3$ | H | $OCH_3$ | 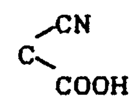 | 134 |
| 8 | O | H | H | H | H | $OCH_3$ | H | $OCH_3$ | 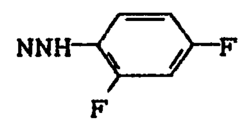 | 171 |
| 9 | O | H | H | H | H | $OCH_3$ | H | $OCH_3$ | 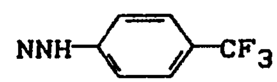 | 170 |
| 10 | O | H | H | H | H | $OCH_3$ | H | $OCH_3$ | 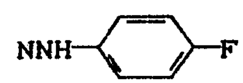 | 145 |

<u>Tabelle 2</u> - Fortsetzung

| Bsp.-Nr | Q | $R^1$ | $R^2$ | $R^3$ | $R^4$ | $R^5$ | $R^6$ | $R^7$ | Z | Schmelz-punkt (°C) |
|---|---|---|---|---|---|---|---|---|---|---|
| 11 | O | H | H | H | H | $OCH_3$ | H | $OCH_3$ | 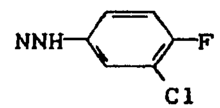 | 187 |
| 12 | O | H | H | H | H | $OCH_3$ | H | $OCH_3$ | 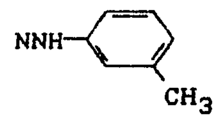 | (amorph) |
| 13 | O | H | H | H | H | $OCH_3$ | H | $OCH_3$ | 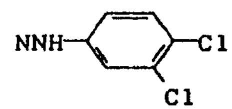 | 174 |
| 14 | O | H | H | H | H | $OCH_3$ | H | $OCH_3$ | 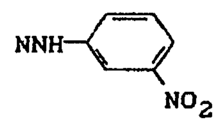 | 185 |
| 15 | O | H | H | H | H | $OCH_3$ | H | $OCH_3$ | 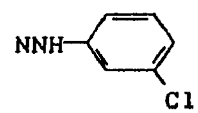 | 171 |
| 16 | O | H | H | H | H | $OCH_3$ | H | $OCH_3$ | 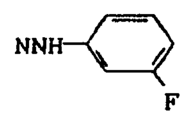 | 149 |

<u>Tabelle 2</u> - Fortsetzung

| Bsp.-Nr | Q | $R^1$ | $R^2$ | $R^3$ | $R^4$ | $R^5$ | $R^6$ | $R^7$ | Z | Schmelzpunkt (°C) |
|---|---|---|---|---|---|---|---|---|---|---|
| 17 | O | H | H | H | H | $OCH_3$ | H | $OCH_3$ | 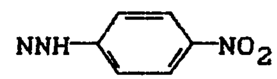 | 235 |
| 18 | O | H | H | H | H | $OCH_3$ | H | $OCH_3$ | 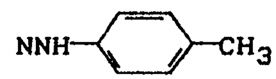 | 123 |
| 19 | O | H | H | H | H | $OCH_3$ | H | $OCH_3$ | 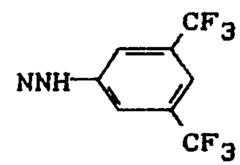 | 178 |
| 20 | O | H | H | H | H | $OCH_3$ | H | $OCH_3$ | 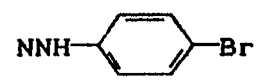 | 141 |
| 21 | O | H | H | H | H | $OCH_3$ | H | $OCH_3$ | 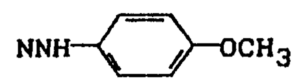 | 141 |
| 22 | O | H | H | H | H | $OCH_3$ | H | $OCH_3$ | 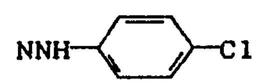 | 147 |

<u>Tabelle 2</u> - Fortsetzung

| Bsp.-Nr | Q | $R^1$ | $R^2$ | $R^3$ | $R^4$ | $R^5$ | $R^6$ | $R^7$ | Z | Schmelzpunkt (°C) |
|---|---|---|---|---|---|---|---|---|---|---|
| 23 | O | H | H | H | H | $OCH_3$ | H | $OCH_3$ | 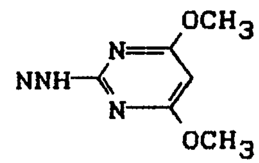 | 160 |
| 24 | O | H | H | H | H | $OCH_3$ | H | $OCH_3$ | 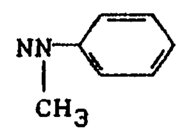 | (amorph) |
| 25 | O | H | H | H | H | $OCH_3$ | H | $OCH_3$ | 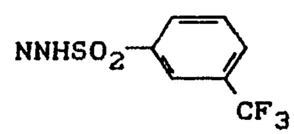 | 168 |
| 26 | O | H | H | H | H | $OCH_3$ | H | $OCH_3$ | 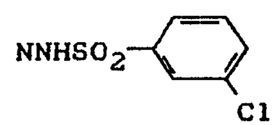 | 175 |
| 27 | O | H | H | H | H | $OCH_3$ | H | $OCH_3$ | 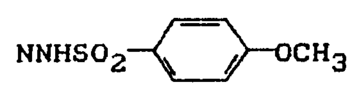 | 145 |

<u>Tabelle 2</u> - Fortsetzung

| Bsp.-Nr | Q | $R^1$ | $R^2$ | $R^3$ | $R^4$ | $R^5$ | $R^6$ | $R^7$ | Z | Schmelz-punkt (°C) |
|---|---|---|---|---|---|---|---|---|---|---|
| 28 | O | H | H | H | H | $OCH_3$ | H | $OCH_3$ | $NNHSO_2$ 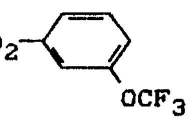 | 129 |
| 29 | O | H | H | H | H | $OCH_3$ | H | $OCH_3$ | $NNHSO_2$ 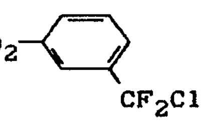 | 112 |
| 30 | O | H | H | H | H | $OCH_3$ | H | $OCH_3$ | $NNHSO_2$ 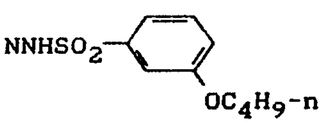 | 134 |
| 31 | O | H | H | H | H | $OCH_3$ | H | $OCH_3$ | $NN(C_6H_5)_2$ | 92 |
| 32 | O | H | H | H | H | $OCH_3$ | H | $OCH_3$ | $NOCHCOOCH_3$<br>\| $CH_3$ | (amorph) |
| 33 | O | H | H | H | H | $OCH_3$ | H | $OCH_3$ | $NNHSO_2CH_3$ | 134 |

<u>Tabelle 2</u> - Fortsetzung

| Bsp.-Nr | Q | $R^1$ | $R^2$ | $R^3$ | $R^4$ | $R^5$ | $R^6$ | $R^7$ | Z | Schmelzpunkt (°C) |
|---|---|---|---|---|---|---|---|---|---|---|
| 34 | O | H | H | H | H | $OCH_3$ | H | $OCH_3$ | NNH 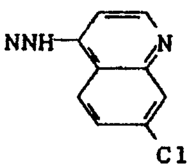 | 196 |
| 35 | O | H | H | H | H | $OCH_3$ | H | $OCH_3$ | $NNHSO_2$ 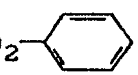 | 65 |
| 36 | O | H | H | H | H | $OCH_3$ | H | $OCH_3$ | $NOCH_2COOC_2H_5$ | (amorph) |
| 37 | O | H | H | H | H | $OCH_3$ | H | $OCH_3$ | $NNHSO_2$ 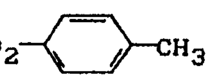 | 170 |
| 38 | O | H | H | H | H | $OCH_3$ | H | $OCH_3$ | $NNHCOCH_3$ | 205 |
| 39 | O | H | H | H | H | $OCH_3$ | H | $OCH_3$ | NNHCO 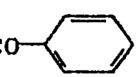 | 179 |
| 40 | O | H | H | H | H | $OCH_3$ | H | $OCH_3$ | NNHCO 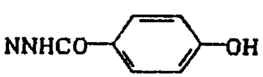 | 215 |

Tabelle 2 - Fortsetzung

| Bsp.-Nr | Q | $R^1$ | $R^2$ | $R^3$ | $R^4$ | $R^5$ | $R^6$ | $R^7$ | Z | Schmelzpunkt (°C) |
|---|---|---|---|---|---|---|---|---|---|---|
| 41 | O | H | H | H | H | $OCH_3$ | H | $OCH_3$ | 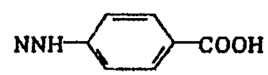 | 240 |
| 42 | O | H | H | H | H | $OCH_3$ | H | $OCH_3$ | 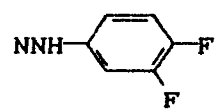 | 174 |
| 43 | O | H | H | H | H | $OCH_3$ | H | $OCH_3$ | 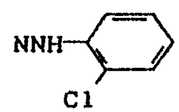 | 161 |
| 44 | O | H | H | H | H | $OCH_3$ | H | $OCH_3$ | 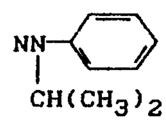 | 113 |
| 45 | O | H | H | H | H | $OCH_3$ | H | $OCH_3$ | 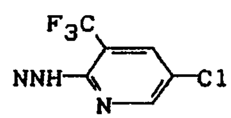 | 214 |
| 46 | O | H | H | H | H | $OCH_3$ | H | $OCH_3$ | $NNHCONH_2$ | 216 |

Tabelle 2 - Fortsetzung

| Bsp.-Nr | Q | $R^1$ | $R^2$ | $R^3$ | $R^4$ | $R^5$ | $R^6$ | $R^7$ | Z | Schmelzpunkt (°C) |
|---|---|---|---|---|---|---|---|---|---|---|
| 47 | O | H | H | H | H | $OCH_3$ | H | $OCH_3$ | 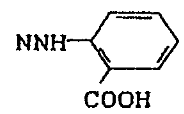 | 219 |
| 48 | O | H | H | H | H | $OCH_3$ | H | $OCH_3$ | 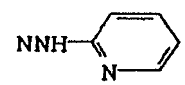 | 142 |
| 49 | O | H | H | H | H | $OCH_3$ | H | $OCH_3$ | 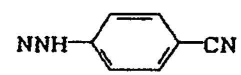 | 224 |
| 50 | O | H | H | H | H | $OCH_3$ | H | $OCH_3$ | 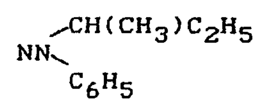 | 89 |
| 51 | O | H | H | H | H | $OCH_3$ | H | $OCH_3$ | 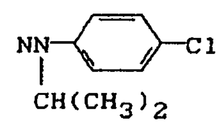 | 95 |
| 52 | O | H | H | H | H | $OCH_3$ | H | $OCH_3$ | 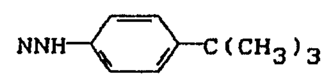 | (amorph) |

Ausgangsstoffe der Formel (II):

Beispiel (II-1)

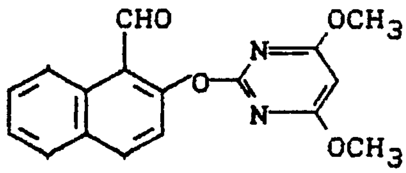

Zu 4,6-Dimethoxy-2-methylsulfonylpyrimidin (6,5 g, 90 mmol) in Acetonitril (100 ml) gibt man 2-Hydroxy-1-naphthaldehyd (5,1 g, 90 mmol) sowie Kaliumcarbonat (8,3 g). Es wird 18 Stunden unter Rückfluß zum Sieden erhitzt, in Eiswasser gegossen und mit Diethylether extrahiert. Nach dem Trocknen über Natriumsulfat wird eingeengt. Die zurückbleibende Substanz wird durch Chromatographie an Silica-Gel gereinigt.

Man erhält 4,7 g (50% der Theorie) 2-(4,6-Dimethoxy-pyrimidin-2-yl-oxy)-1-naphthaldehyd vom Schmelzpunkt 135° C.

Ausgangsstoffe der Formel (IV):

Beispiel (IV-1)

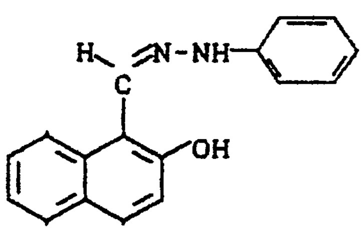

Zu 2-Hydroxy-1-naphthaldehyd (8,6 g, 50 mmol) in Toluol (70 ml) gibt man Phenylhydrazin (5,4 g, 50 mmol) und rührt 1 h bei 20° C nach. Das kristalline Produkt wird durch Absaugen isoliert.

Man erhält 2-Hydroxy-1-naphthaldehyd-phenylhydrazon (9,6 g, 73 % der Theorie) vom Schmelzpunkt 210° C (Zers.).

Anwendungsbeispiele:

In den Anwendungsbeispielen wird die nachstehend skizzierte Verbindung (A) als Vergleichssubstanz herangezogen:

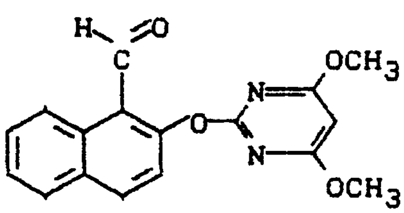

(A)

2-(4,6-Dimethoxy-pyrimidin-2-yl-oxy)-1-naphthaldehyd (bekannt aus EP-A 360 163 und JP-A 2 056 469).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß folgender Herstellungsbeispiele: 1, 8, 9, 10, 11, 12, 13, 14, 15, 16, 19, 21, 27, 28, 31 und 33.

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß folgender Herstellungsbeispiele: 1, 7, 8, 9, 10, 12, 13, 14, 15, 16, 19, 20, 21, 22, 24, 25, 26, 27, 29, 30, 31 und 33.

## Patentansprüche

**1.** Naphthalinderivate der allgemeinen Formel (I)

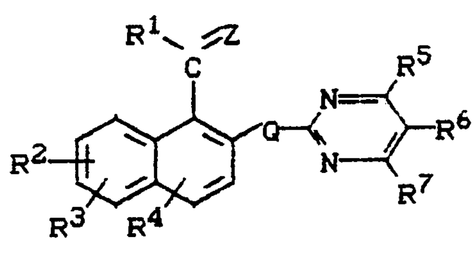

(I)

in welcher

| | |
|---|---|
| Q | für Sauerstoff, Schwefel oder die Gruppierung NR steht, wobei R für Wasserstoff oder Alkyl steht, |
| $R^1$ | für Wasserstoff oder Alkyl steht, |
| $R^2$, $R^3$ und $R^4$ | gleich oder verschieden sind und jeweils für Wasserstoff, Hydroxy, Amino, Cyano, |

Nitro, Halogen oder für jeweils gegebenenfalls substituiertes Alkyl, Alkoxy, Alkylthio, Alkylsulfinyl oder Alkylsulfonyl stehen,

$R^5$ für Wasserstoff, Halogen oder für jeweils gegebenenfalls substituiertes Alkyl, Alkoxy, Alkylthio, Alkylamino oder Dialkylamino steht,

R $^6$ für Wasserstoff, Halogen oder für jeweils gegebenenfalls substituiertes Alkyl oder Alkoxy steht und

$R^7$ für Wasserstoff, Halogen oder für jeweils gegebenenfalls substituiertes Alkyl, Alkoxy, Alkylthio, Alkylamino oder Dialkylamino steht, und

Z für eine der nachstehenden Gruppierungen steht: N-$R^8$ oder

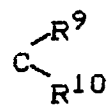 ,

wobei

$R^8$ für Wasserstoff, Hydroxy, Amino, Carbamoylamino oder für jeweils gegebenenfalls substituiertes Alkyl, Alkenyl, Alkinyl, Alkoxy, Alkenyloxy, Alkoxycarbonylalkoxy, Alkylamino, Dialkylamino, Alkylcarbonylamino, Alkoxycarbonylamino, Alkylsulfonylamino, Aryl, Aralkyl, Aryloxy, Aralkyloxy, Arylamino, Diarylamino, Aralkylamino, N-Alkyl-N-arylamino, Hetarylamino, Hetarylcarbonylamino, Arylcarbonylamino oder Arylsulfonylamino steht,

$R^9$ für Wasserstoff, Halogen, Cyano, Carboxy, Alkoxycarbonyl, Alkylcarbonylamino oder Dialkoxyphosphoryl steht und

$R^{10}$ für Formyl, Cyano, Carboxy, Hydroxymethyl, Carbamoyl oder für jeweils gegebenenfalls substituiertes Alkoxycarbonyl, Cycloalkoxycarbonyl, Alkylthiocarbonyl, Alkylaminocarbonyl, Cycloalkylaminocarbonyl, Dialkylaminocarbonyl, Alkylaminocarbonylalkoxycarbonyl, Dialkylaminocarbonylalkoxycarbonyl, Arylaminocarbonylalkoxycarbonyl, N-Alkyl-N-arylaminocarbonylalkoxycarbonyl, Pyrrolidinylcarbonyl, Piperidinylcarbonyl, Morpholinylcarbonyl, Piperazinylcarbonyl, Aryloxycarbonyl, Aralkyloxycarbonyl, Heterocyclylalkoxycarbonyl, Arylthiocarbonyl, Aralkylthiocarbonyl, Arylaminocarbonyl, Aralkylaminocarbonyl, N-Alkyl-N-arylaminocarbonyl, Arylhydrazinocarbonyl, Alkylhydrazinocarbonyl oder Phthalimidoxycarbonyl steht oder $R^{10}$ zusammen mit $R^9$ für die Gruppierung $-CO-O(CH_2)_n-$steht, wobei n für die Zahlen 1 bis 4 steht.

**2.** Naphthalinderivate der allgemeinen Formel (I) gemäß Anspruch 1, dadurch gekennzeichnet, daß darin

Q für Sauerstoff, Schwefel oder die Gruppierung NR steht, wobei R für Wasserstoff oder $C_1$-$C_4$-Alkyl steht,

$R^1$ für Wasserstoff oder $C_1$-$C_4$-Alkyl steht,

$R^2$, $R^3$ und $R^4$ gleich oder verschieden sind und jeweils für Wasserstoff, Hydroxy, Amino, Cyano, Nitro, Halogen oder für jeweils gegebenenfalls durch Halogen substituiertes $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl stehen,

$R^5$ für Wasserstoff, Halogen oder für jeweils gegebenenfalls durch Halogen oder $C_1$-$C_4$-Alkoxy substituiertes $C_1$-$C_4$-Alkyl, $C_1$-$C_4$-Alkoxy, $C_1$-$C_4$-Alkylthio, $C_1$-$C_4$-Alkylamino oder Di-($C_1$-$C_4$-alkyl)-amino steht,

$R^6$ für Wasserstoff, Halogen oder für jeweils gegebenenfalls durch Halogen substituiertes $C_1$-$C_4$-Alkyl oder $C_1$-$C_4$-Alkoxy steht,

$R^7$ für Wasserstoff, Halogen oder für jeweils gegebenenfalls durch Halogen substituiertes $C_1$-$C_4$-Alkyl, $C_1$-$C_4$-Alkoxy, $C_1$-$C_4$-Alkylthio, $C_1$-$C_4$-Alkylamino oder Di-($C_1$-$C_4$-alkyl)-amino steht,

Z für eine der nachstehenden Gruppierungen steht: N-$R^8$ oder

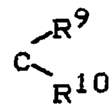 ,

wobei

$R^8$ für Wasserstoff, Hydroxy, Amino, Carbamoylamino oder für jeweils gegebenenfalls durch Halogen substituiertes $C_1$-$C_6$-Alkyl, $C_3$-$C_6$-Alkenyl, $C_3$-$C_6$-Alkinyl, $C_1$-$C_6$-Alkoxy, $C_3$-$C_6$-Alkenyloxy, $C_1$-$C_4$-Alkoxy-carbonyl-$C_1$-$C_2$-alkoxy, $C_1$-$C_6$-Alkylamino, Di-($C_1$-$C_2$-alkyl)-amino, $C_1$-$C_6$-Alkyl-carbonylamino, $C_1$-$C_6$-Alkoxy-carbonylamino, $C_1$-$C_6$-Alkyl-sulfonylamino, oder für jeweils gegebenenfalls durch Nitro, Hydroxy, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituiertes Phenyl, Phenyl-$C_1$-$C_4$-alkyl, Phenoxy, Phenyl-$C_1$-$C_4$-alkoxy, Phenylamino, Diphenylamino, Phenyl-$C_1$-$C_4$-alkylamino, N-($C_1$-$C_4$-alkyl)-N-phenylamino, Pyridylamino, Pyrimidylamino, Chinolylamino, Pyridylcarbonylamino, Phenylcarbonylamino, Furylcarbonylamino, Thienylcarbonylamino oder Phenylsulfonylamino steht,

$R^9$ für Wasserstoff, Halogen, Cyano, Carboxy, $C_1$-$C_6$-Alkoxy-carbonyl, $C_1$-$C_6$-Alkylcarbonylamino oder Di-($C_1$-$C_4$-alkoxy)-phosphoryl steht und

$R^{10}$ für Formyl, Cyano, Carboxy, Hydroxymethyl, Carbamoyl oder für jeweils gegebenenfalls durch Halogen, Carboxy oder $C_1$-$C_4$-Alkoxy-carbonyl substituiertes $C_1$-$C_6$-Alkoxy-carbonyl, $C_5$-$C_6$-Cycloalkyloxy-carbonyl, $C_1$-$C_6$-Alkylthio-carbonyl, $C_1$-$C_6$-Alkylamino-carbonyl oder $C_5$-$C_6$-Cycloalkylamino-carbonyl, für Di-($C_1$-$C_2$-alkyl)-amino-carbonyl, für $C_1$-$C_4$-Alkylamino-carbonyl-$C_1$-$C_4$-alkoxy-carbonyl, für Di-($C_1$-$C_2$-alkyl)-amino-carbonyl-$C_1$-$C_4$-alkoxy-carbonyl, für Phenylaminocarbonyl-$C_1$-$C_4$-alkoxy-carbonyl, für N-Methyl-N-phenylaminocarbonyl-$C_1$-$C_4$-alkoxy-carbonyl, für jeweils gegebenenfalls durch Methyl und/oder Ethyl substituiertes Pyrrolidinylcarbonyl, Piperidinyl-carbonyl, Morpholinylcarbonyl oder Piperazinylcarbonyl, für jeweils gegebenenfalls durch Nitro, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituiertes Phenoxycarbonyl, Phenyl-$C_1$-$C_4$-alkoxycarbonyl, Furylmethoxycarbonyl, Thienylmethoxycarbonyl, Phenylthiocarbonyl, Phenyl-$C_1$-$C_4$-alkylthio-carbonyl, Phenylaminocarbonyl, Phenyl-$C_1$-$C_4$-alkylamino-carbonyl, N-($C_1$-$C_4$-Alkyl)-N-phenylamino-carbonyl oder Phenylhydrazinocarbonyl, für $C_1$-$C_4$-Alkylhydrazinocarbonyl oder für Phthalimidoxycarbonyl steht oder $R^{10}$ zusammen mit $R^9$ für die Gruppierung $-CO-O-(CH_2)_n-$ steht, wobei

n für die Zahlen 1 bis 4, insbesondere 2 oder 3 steht.

3. Naphthalinderivate der allgemeinen Formel (I) gemäß Anspruch 1, dadurch gekennzeichnet, daß darin

Q für Sauerstoff steht,

$R^1$ für Wasserstoff steht,

$R^2$, $R^3$ und $R^4$ für Wasserstoff stehen,

$R^5$ für Wasserstoff, Chlor, Methyl, Ethyl, Trifluormethyl, Methoxymethyl, Methoxy, Ethoxy, Difluormethoxy, Methylthio, Methylamino, Ethylamino oder Dimethylamino - insbesondere für Methoxy - steht,

$R^6$ für Wasserstoff, Chlor, Methyl oder Methoxy - insbesondere für Wasserstoff - steht, und

$R^7$ für Wasserstoff, Chlor, Methyl, Ethyl, Trifluormethyl, Methoxy, Ethoxy, Difluormethoxy, Methylthio, Methylamino, Ethylamino oder Dimethylamino - insbesondere für Methoxy - steht, und

Z für eine der nachstehenden Gruppierungen steht: N-$R^8$ oder

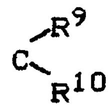 ,

wobei

$R^8$ für Wasserstoff, Hydroxy, Amino, Carbamoylamino, für Methyl, Ethyl, n-Propyl, Isopropyl, n-Butyl, Isobutyl, sec-Butyl, tert-Butyl, Allyl, Propargyl, Methoxy, Ethoxy, n-Propoxy, Isopropoxy, n-Butoxy, Isobutoxy, sec-Butoxy, Allyloxy, Methoxycarbonylmethoxy, Ethoxycarbonylmethoxy, Methoxycarbonylethoxy, Ethoxycarbonylethoxy, Methylamino, Ethylamino, n-Propylamino, Isopropylamino, n-Butylamino, Isobutylamino, sec-Butylamino, tert-Butylamino, Dimethylamino, Acetylamino, Propionylamino, Methoxycarbonylamino, Ethoxycarbonylamino, Methylsulfonylamino, Ethylsulfonylamino, für jeweils gegebenenfalls durch Nitro, Hydroxy, Cyano, Carboxy, Fluor, Chlor, Brom, Methyl, Trifluormethyl, Methoxy, Trifluormethoxy, Methylthio oder Trifluormethylthio substituiertes Phenyl, Benzyl, Phenoxy, Benzyloxy, Phenylamino, Diphenylamino, Benzylamino, N-Methyl-N-phenylamino, Pyridylamino, Pyrimidylamino, Chinolylamino, Pyridylcarbonylamino, Phenylcarbonylamino, Furylcarbonylamino, Thienylcarbonylamino oder Phenylsulfonylamino steht,

$R^9$ für Wasserstoff, Fluor, Chlor, Cyano, Carboxy, $C_1$-$C_4$-Alkoxy-carbonyl, $C_1$-$C_4$-Alkyl-carbonylamino, Dimethoxyphosphoryl oder Diethoxyphosphoryl steht und

$R^{10}$ für Formyl, Cyano, Carboxy, Hydroxymethyl, Carbamoyl, für jeweils gegebenenfalls durch Fluor, Chlor, Carboxy oder $C_1$-$C_4$-Alkoxy-carbonyl substituiertes $C_1$-$C_4$-Alkoxy-carbonyl, $C_5$-$C_6$-Cycloalkyloxy-carbonyl, $C_1$-$C_4$-Alkylthiocarbonyl, $C_1$-$C_4$-Alkylamino-carbonyl oder $C_5$-$C_6$-Cycloalkylamino-carbonyl, für Dimethylaminocarbonyl, für $C_1$-$C_4$-Alkylaminocarbonyl-$C_1$-$C_4$-alkoxy-carbonyl, für Dimethylaminocarbonyl-$C_1$-$C_4$-alkoxy-carbonyl, für N-Methyl-N-phenylaminocarbonyl-$C_1$-$C_4$-alkoxy-carbonyl, für jeweils gegebenenfalls durch Methyl und/oder Ethyl substituiertes Pyrrolidinyl-carbonyl, Piperidinyl-carbonyl, Morpholinyl-carbonyl oder Piperazinyl-carbonyl, für jeweils gegebenenfalls durch Nitro, Cyano, Fluor, Chlor, Brom, Methyl, Trifluormethyl, Methoxy, Trifluormethoxy, Methylthio oder Trifluormethylthio substituiertes Phenoxycarbonyl, Benzyloxycarbonyl, Phenylthiocarbonyl, Benzylthiocarbonyl, Phenylaminocarbonyl, Benzylaminocarbonyl, N-Methyl-N-phenylaminocarbonyl, Phenylhydrazinocarbonyl oder für Phthalimidoxycarbonyl steht oder $R^{10}$ zusammen mit $R^9$ für die Gruppierung $-CO-O-CH_2CH_2-$steht.

**4.** Verfahren zur Herstellung von Naphthalinderivaten der Formel (I) gemäß Anspruch 1, dadurch gekennzeichnet, daß man

(a) entsprechende Carbonylverbindungen der allgemeinen Formel (II)

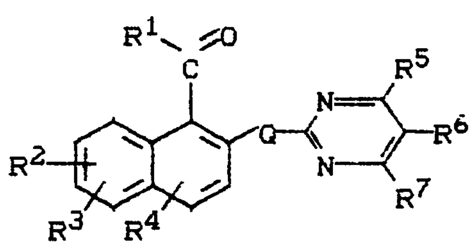 (II)

in welcher

Q, $R^1$, $R^2$, $R^3$, $R^4$, $R^5$, $R^6$ und $R^7$ die in Anspruch 1 angegebene Bedeutung haben,

mit Amino- oder Methylen-Verbindungen der allgemeinen Formel (III),

$H_2Z$ (III)

in welcher

Z die in Anspruch 1 angegebene Bedeutung hat, gegebenenfalls in Gegenwart eines Reaktionshilfsmittels und gegebenenfalls in Gegenwart eines Verdünnungsmittels umsetzt, oder daß man

(b) nucleophile Naphthalinderivate der allgemeinen Formel (IV),

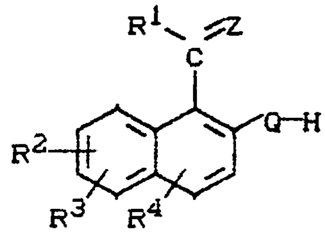

(IV)

in welcher

Q, $R^1$ $R^2$, $R^3$, $R^4$ und Z die in Anspruch 1 angegebene Bedeutung haben,

mit Pyrimidinderivaten der allgemeinen Formel (V),

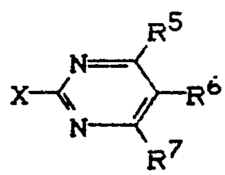

(V)

in welcher

$R^5$, $R^6$ und $R^7$ die in Anspruch 1 angegebene Bedeutung haben und

X für eine nucleofuge Abgangsgruppe steht,

gegebenenfalls in Gegenwart eines Säureakzeptors und gegebenenfalls in Gegenwart eines Verdünnungsmittels umsetzt.

5. Herbizide Mittel, gekennzeichnet durch einen Gehalt an mindestens einem Naphthalinderivat der allgemeinen Formel (I) gemäß Anspruch 1.

6. Verfahren zur Bekämpfung von Unkraut, dadurch gekennzeichnet, daß man ein Naphthalinderivat der Formel (I) gemäß Anspruch 1 auf die Unkräuter oder ihren Lebensraum einwirken läßt.

7. Verwendung von Naphthalinderivaten der Formel (I) gemäß Anspruch 1 zur Bekämpfung von unerwünschtem Pflanzenwachstum.

8. Verfahren zur Herstellung von herbiziden Mitteln, dadurch gekennzeichnet, daß man Naphthalinderivate der Formel (I) gemäß Anspruch 1 mit Streckmitteln und/oder oberflächenaktiven Stoffen vermischt.